(19) Europäisches Patentamt
European Patent Office
Office européen des brevets

(11) Publication number: **0 636 633 A1**

## (12) EUROPEAN PATENT APPLICATION

(21) Application number: **93305973.5**

(51) Int. Cl.⁶: **C07J 63/00**, A61K 31/58

(22) Date of filing: **28.07.93**

(43) Date of publication of application: **01.02.95 Bulletin 95/05**

(84) Designated Contracting States: **CH DE FR GB IT LI NL SE**

(71) Applicant: **KOWA CHEMICAL INDUSTRIES CO., LTD.**
**No. 3-13, Minami 6-chome, Hounan-cho**
**Toyonaka-shi, Osaka (JP)**

(72) Inventor: **Atsuchi, Mikito**
**1-23-2, Chidori**
**Ota-ku, Tokyo (JP)**

Inventor: **Yamashita, Chiaki**
**1-27-4, Shimorenjaku**
**Mitaka-shi, Tokyo (JP)**

Inventor: **Iwasaki, Yoshio**
**2-30-10, Sanno**
**Ota-ku, Tokyo (JP)**

(74) Representative: **Jones, Helen Marjorie Meredith**
**Gill Jennings & Every,**
**Broadgate House,**
**7 Eldon Street**
**London EC2M 7LH (GB)**

## (54) A triterpenoid saponin, extraction thereof and use to treat or prevent diabetes mellitus.

(57) A novel triterpenoid saponin having neither unpleasant tastes, such as astringency and bitterness, nor inhibitory effect on the sweet taste responses but having an inhibitory effect on glucose absorption, a preventive and therapeutic agent for diabetes mellitus, an additive to a diet and a diet containing the triterpenoid saponin.

The triterpenoid saponin comprises (3β,16β)-16,28-dihydroxyolean-12-en-3-yl-2-O-β-D-glucopyranosyl-β-D-glucopyranosiduronic acid. The intended triterpenoid saponin can be provided by subjecting leaves of Gymnema inodorum widely distributed in Southeast Asia to extraction with a solvent, evaporating the extract to dryness, washing the residue with an acid to remove base ingredients, defatting the washed residue, extracting the defatted residue with acetone, evaporating the extract to dryness, extracting the residue with diethyl carbonate to provide a crude crystal, dissolving the crude crystal in methanol, collecting a fraction eluted in a period between 31.0 min and 33.0 min by HPLC, and recrystallizing the fraction from a solvent of acetone/chloroform (50/50). A therapeutic agent for diabetes mellitus comprising as an active ingredient the triterpenoid saponin thus obtained, and a diet comprising as an additive the triterpenoid saponin are also provided.

*Fig. 2*

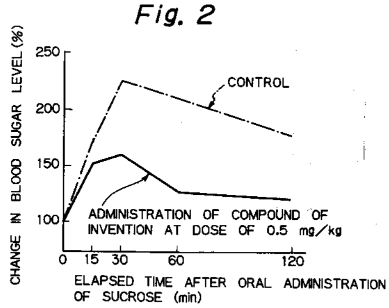


EP 0 636 633 A1

## BACKGROUND OF THE INVENTION

### 1. Field of the Invention

The present invention relates to a novel triterpenoid saponin having no unpleasant taste, such as astringency, and substantially no inhibitory effect on the sweet taste responses but having an inhibitory effect on glucose absorption and a process for producing the same.

Further, the present invention relates to a preventive and therapeutic agent for diabetes mellitus.

Further, the present invention relates to a diet containing a triterpenoid saponin that is a novel compound having an inhibitory effect on glucose absorption.

### 2. Description of the Related Art

Phlorizin, Gymnemic acid and Ziziphin are known as substances having an inhibitory effect on glucose absorption. However, these substances had a problem regarding use because Gymnemic acid and Ziziphin have an inhibitory effect on the sweet taste responses and, in particular, Gymnemic acid has bitterness.

The present inventors have previously found that leaves of a plant called "Gymnema inodorum" widely distributed in Southeast Asia contain a substance that has no astringency, bitterness and inhibitory effect on the sweet taste responses but has an inhibitory effect on glucose absorption, and proposed an inhibitor for the absorption of glucose that is in the form of a tea prepared by drying and roasting the leaves of Gymnema inodorum or in the form of tablets or granules prepared by subjecting the leaves of Gymnema inodorum to extraction with hot water, an alcohol or the like and concentrating or drying the extract (see Japanese Unexamined Patent Publication (Kokai) No. 3-172156).

Thereafter, the present inventors have made extensive and intensive studies on a method for separating and purifying an active ingredient having an inhibitory effect on glucose absorption from the above-described Gymnema inodorum and, as a result, have succeeded in separating and purifying a triterpenoid saponin having no astringency, bitterness and inhibitory effect on the sweet taste responses but having an inhibitory effect on glucose absorption, and have found that the triterpenoid saponin is useful as a preventive and therapeutic agent for diabetes mellitus.

Further, the present inventors have newly found that the addition of the active ingredient to diets enables the absorption of glucose to be effectively inhibited.

Thereafter, the present inventors have made extensive and intensive studies on a method for separating and purifying an active ingredient having no astringency, bitterness and inhibitory effect on the sweet taste responses but having an inhibitory effect on glucose absorption from leaves of the above-described Gymnema inodorum, and, as a result, have succeeded in extracting and isolating the active ingredient.

Accordingly, an object of the present invention is to provide the above-described active ingredient and a process for producing the same.

Another object of the present invention is to provide a preventive and therapeutic agent for diabetes mellitus, comprising the above-described triterpenoid saponin as an active ingredient.

A further object of the present invention is to provide a diet that imparts neither astringency nor bitterness and has an inhibited absorption of glucose.

## SUMMARY OF THE INVENTION

According to one aspect of the present invention, there is provided a triterpenoid saponin represented by the following general formula I:

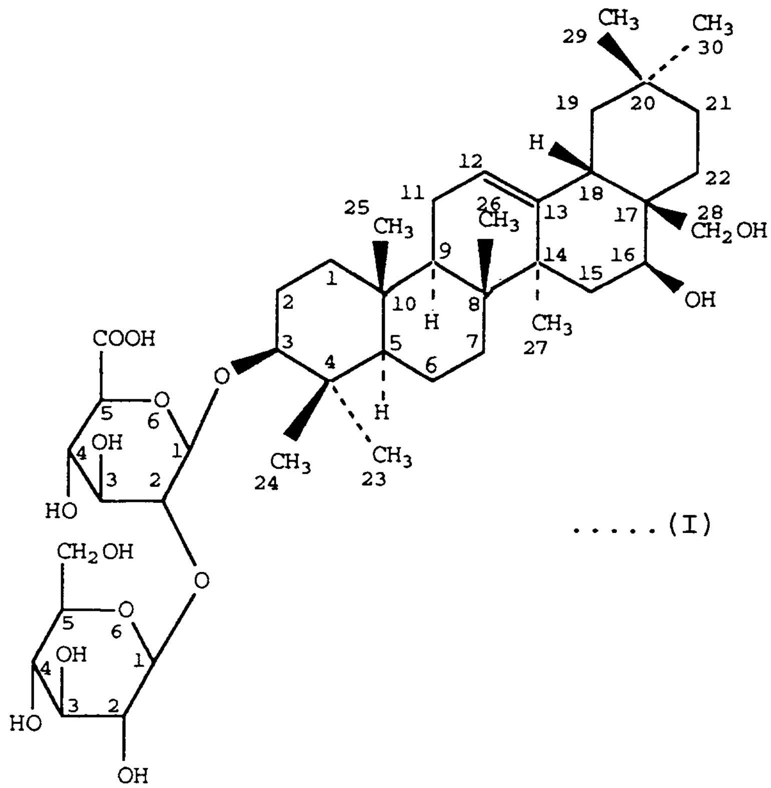

..... (I)

According to another aspect of the present invention, there is provided a process for producing a triterpenoid saponin represented by the above-described general formula I, comprising subjecting leaves of Gymnema inodorum to extraction with a solvent, filtering the extract, evaporating the filtrate to dryness, washing the residue with petroleum ether, extracting the washed residue with acetone, filtering the extract, evaporating the filtrate to dryness, extracting the residue with diethyl carbonate, and precipitating a crystal from the extract.

According to a further aspect of the present invention, there is provided a preventive or therapeutic agent for diabetes mellitus, comprising as an active ingredient a triterpenoid saponin represented by the above-described general formula (I).

According to a further aspect of the present invention, there is provided a diet containing a triterpenoid saponin represented by the above-described general formula (I).

## BRIEF DESCRIPTION OF THE DRAWINGS

Fig. 1 is a diagram showing a fraction chart of HPLC for a crude crystal provided in Example 1; and
Fig. 2 is a diagram showing the inhibitory effect of the triterpenoid saponin of the present invention on glucose absorption.

## DESCRIPTION OF PREFERRED EMBODIMENTS

The present invention will now be described in detail.

The triterpenoid saponin of the present invention can be produced by subjecting leaves of Gymnema inodorum, which is a plant belonging to Asclepiadaceae growing naturally in India, Myanma, Thai, Malaysia, Indonesia, Vietnam and China to extraction with water, methanol or a mixed solvent, such as water/methanol or water/ethanol, subjecting the extract to rough purification and subjecting the crude product to isolation and purification by chromatography, such as liquid chromatography.

The Gymnema inodorum used as a raw material in the present invention is described in Flore Generale de L' Indo-China, VI, 87, (1930), Illustrated Book of the Chinese Higher Flora, Vol. 3, 495, (1974), Thai Plant Names, 169, Florae Siamensis Enumeratio, III, 21 (1951), etc.

Examples of aliases for Gymnema inodorum include Kwang Tung Geng Shi Peng (see Illustrated Book of the Chinese Higher Flora, Vol. 3, 495, (1974)), Cynanchum inodorum (Fl. Cochinch., 166 (1790), C. retic

ulatum (Obs. fasc. 2., 15), Bidaria inodora (Fl. Brit. Ind., IV, 33), Gymnema tingens (Fl. Brit. Ind., IV, 31, etc.) and Asclepias tingens (Hort. Beng., 21 (1841)).

The triterpenoid saponin as the active ingredient of the present invention is (3$\beta$,16$\beta$)-16,28-dihydroxyolean-12-en-3-yl-2-O-$\beta$-D-glucopyranosyl-$\beta$-D-glucopyranosiduronic acid represented by the above-described general formula (I) and can be produced by subjecting leaves of Gymnema inodorum belonging to Asclepiadaceae widely distributed in Southeast Asia to extraction with water, methanol or a mixed solvent, such as water/methanol or water/ethanol, subjecting the extract to rough purification and subjecting the crude product to isolation and purification by chromatography, such as liquid chromatography.

The above-described triterpenoid saponin is a physiologically active substance that inhibits the absorption of glucose through intestines and prevents a rapid increase in the blood sugar level, has neither astringency nor bitterness and is useful as a preventive and therapeutic agent for diabetes mellitus. In particular, since it can inhibit the absorption of glucose, a synergistic effect can be advantageously attained when it is used in combination with a restricted diet.

The preventive and therapeutic agent for diabetes mellitus according to the present invention may be mixed with or supported on carriers used in the production of medicines and orally administered in the form of powders, granules, tablets or liquid preparations such as capsules or syrups, suspensions, liquid preparations and emulsions. Other ingredients, such as vitamins, may be incorporated therein without any problem.

The agent may be administered at a dose of 0.1 to 100 mg/kg/time and 2 to 3 times/day. It is a matter of course that the dose and number of times of administration may vary depending upon the severity of disease of the patient.

The above-described triterpenoid saponin is a physiologically active compound that inhibits the absorption of glucose through intestines and prevents a rapid increase in the blood sugar level. Therefore, when the compound is added to diets, since it inhibits the absorption of glucose formed by digestion of glucide contained in the diets, the diets containing the above-described triterpenoid saponin are favorable as health foods and drinks or foods and drinks for patients suffering from diabetes mellitus for whom an increase in the blood sugar level is unfavorable.

The diets contemplated in the present invention include not only various juices, candies and chocolates but also all kinds of confectionery and ice creams and foods and drinks containing glucide, such as noodles, rice, rice cake, sweeteners, jams and liquors. Further, since the inhibitory effect of the triterpenoid saponin on glucose absorption persists for 2 to 3 hr, the triterpenoid saponin can be added to diets not containing glucide, for example, potable water, tea and pickles. Further, it is also possible to add the triterpenoid saponin to seasonings, such as soy sauce, *miso* and mayonnaise.

Even when the triterpenoid saponin is added in a small amount, the effect can be attained in its own way. In general, the amount of addition of the triterpenoid saponin may be 1 to 300 mg per 50 to 60 g of glucide contained in the diet. It is preferably in the range of from 10 to 90 mg per 50 to 60 g of glucide contained in the diet. Although it may be 300 mg or more per 50 to 60 g of saccharide contained in the diet, since the effect is saturated, the addition in such a large amount is unfavorable from an economical viewpoint.

## EXAMPLES

The present invention will now be described in more detail with reference to the following Examples, though it is not limited to these Examples only.

### Example 1

<u>Extraction</u>

Leaves of Gymnema inodorum were dried at 60 °C for 16 hr and ground into 2-mm cuts. One liter of methanol was added to 100 g of the dried ground leaf thus obtained, and the mixture was subjected to extraction under reflux with stirring for 2 hr, and was then filtered.

<u>Removal of Water-Soluble Base Ingredients</u>

30 g of Celite was added to the filtrate, and the mixture was evaporated to dryness under reduced pressure (80 °C/evaporator). 51.2 g of the residue was washed twice with 1 liter of distilled water regulated to a pH value of 2 with HCl and filtered, and the residue was dried.

Defatting and Rough Crystallization

41.0 g of the dried residue was repeatedly washed twice with 200 cc of petroleum ether, filtered and dried, and 36.8 g of the dried residue was repeatedly extracted twice with 200 cc of acetone at room temperature. The extract was filtered, and the filtrate was evaporated to dryness. 5.3 g of the residue was repeatedly extracted under reflux three times with 100 cc of diethyl carbonate. The extract was filtered with heat reserving. The crystal precipitated from the filtrate was filtered and dried in vacuo to provide 1.4 g of a crude crystal.

Collection of Intended Fraction

Then, the crude crystal was dissolved in methanol to prepare a 10% (W/V) methanol solution, and an intended fraction was collected by preparative HPLC (1 cc x 10 times).

Conditions for HPLC were as follows.

Device for HPLC: Tosoh 8010 series

| | |
|---|---|
| System controller | SC-8010 |
| Column oven | CO-8010 |
| Pump | CCPP-M |
| Fraction collector | FC-8010 etc. |
| Ultraviolet-visible | UV-8010 |

Fractionation column: TSK gel ODS-80TM (21.5 mm ID x 30 cm)
Composition of eluent: $H_2O/CH_3CN/CH_3COOH$ = 50/50/0.1 (isocratic)
Flow rate: 6 ml/min
Detection wavelength: 210 nm
Amount of injection: 1000 $\mu$l (sample concentration: 10%) x 10 times
Collected fraction: 31.0-33.0 min (peak time = 32.02 min
Amount of collection: 105 mg

A fraction chart obtained by HPLC is shown in Fig. 1.

Recrystallization

120 cc of the collected eluate obtained by the above-described step was evaporated to dryness under reduced pressure (150 mg), the residue was heat-dissolved in 10 cc of acetone, and hot chloroform was added to the solution until the solution became slightly cloudy (an amount substantially equal to the amount of acetone). The mixture was again heated in a water bath for complete dissolution. The solution was allowed to stand to precipitate a white crystal which was then filtered and dried in vacuo to provide 58 mg of triterpenoid saponin having a purity of 100.00% (as determined by HPLC) as an intended product.

The properties of the resultant triterpenoid saponin were as follows.

m.p.: 244-246 °C
IR spectrum: 3400 (s), 2950 (s), 1720 (m), 1650 (m), 1450 (m), 1360 (m), 1260 (m), 1205 (w), 1160 (m), 1080 (s), 1050 (s), 950 (w), 925 (w), 900 (w) $cm^{-}$

Solvents useable in the extraction and extraction conditions and extraction efficiency are given in the following Table 1.

Table 1

| Extraction Solvent | Extraction Temp. (°C) | Extraction Time. (hr) | Yield of Extract (%) | Content of Intended Ingredient in Extract (%) | Percentage Extraction of Intended Ingredient (%) |
|---|---|---|---|---|---|
| a) Methanol + 0.1 % pyridine* | Reflux | 2 | 26.6 | 10.61 | 2.822 |
| b) Distilled water/ethanol = 50/50 | 60 | 2 | 36.5 | — | — |
| | | 4 | 36.2 | — | — |
| | | 6 | 39.5 | — | — |
| | Reflux | 2 | 36.0 | 6.85 | 2.466 |
| | | 4 | 36.6 | — | — |
| | | 6 | 39.4 | — | — |
| c) Methanol | Reflux | 2 | 25.1 | 9.43 | 2.367 |
| d) Distilled water/methanol = 50/50 | Reflux | 2 | 33.6 | 6.75 | 2.268 |
| e) Distilled water + 0.1% sodium hydrogencarbonate* | 80 | 2 | 39.1 | 2.98 | 1.165 |
| f) Distilled water | 60 | 2 | 30.9 | — | — |
| | | 4 | 29.9 | — | — |
| | | 6 | 32.0 | — | — |
| | 80 | 2 | 32.3 | 2.91 | 0.940 |
| | | 4 | 34.8 | — | — |
| | | 6 | 36.1 | — | — |
| | Reflux | 2 | 35.8 | — | — |
| | | 4 | 39.4 | — | — |
| | | 6 | 43.7 | — | — |
| g) Ethanol | Reflux | 2 | 13.8 | 6.51 | 0.898 |
| h) i-Propanol | Reflux | 2 | 9.9 | 1.92 | 0.190 |
| i) Acetone | Reflux | 2 | 7.2 | 0.45 | 0.032 |

Note) *: Pyridine and sodium hydrogencarbonate were added to the extraction solvent because the addition of these compounds contributes to an increase in the extraction efficiency. It is also possible to add $Na_2CO_3$.

In the step of removing water-soluble base ingredients, Celite was added to the filtrate for the purpose of preventing the extract from becoming tarry to render the handleability poor and, at the same time, effecting decoloring.

It is also possible to use activated carbon, silica gel, zeolite, etc. besides Celite.

In the step of removing water-soluble base ingredients, HCl was used for the purpose of adjusting the pH value of washing water to 2. It is also possible to use, besides hydrochloric acid, other acids, such as sulfuric acid and acetic acid.

In the step of recrystallization, although a mixed solvent of acetone/chloroform (50/50) is preferred as the solvent, it is also possible to use a mixed solvent of acetone/cyclohexane, acetone/n-hexane methanol/xylene or ethanol/xylene acetone/benzene.

Example 2

Extraction

100 g of the same dried ground leaf as that used in Example 1 was extracted with 1 liter of water/ethanol (50/50) under reflux with stirring for 2 hr, and the extract was filtered.

Removal of Water-Soluble Base Ingredients

30 g of Celite was added to the filtrate, and the mixture was evaporated to dryness under reduced pressure (80°C/evaporator). 63.7 g of the residue was washed twice with 1 liter of an aqueous acetic acid solution having a pH value of 2 and filtered, and the residue was dried.

Defatting and Rough Crystallization

42.3 g of the dried residue was washed in 200 cc of petroleum ether for 2 hr, filtered and dried.

35.6 g of the dried residue was extracted with 200 cc of acetone at room temperature for 2 hr. The extract was filtered, and the filtrate was evaporated to dryness. 5.5 g of the residue was repeatedly extracted under reflux three times with 100 cc of diethyl carbonate. The extract was filtered with heat reserving. The crystal precipitated from the filtrate was filtered and dried in vacuo to provide 1.3 g of a crude crystal.

Collection of Intended Fraction

Then, a 10 % (W/V) methanol solution of the resultant crude crystal was used to effect preparative HPLC in the same manner as that of Example 1. In the preparative HPLC, fractions eluted in a period between 31.0 min and 33.0 min were collected, and 120 cc of the collected eluate was evaporated to dryness under reduced pressure to provide 113 mg of a crystal.

Recrystallization

113 mg of the crystal was recrystallized from 20 cc of an acetone/chloroform solvent in the same manner as that of Example 1, and the crystal was dried in vacuo to provide 72 mg of triterpenoid saponin having a melting point of 244 to 248 °C and a purity of 99.8 % (as determined by HPLC) as an intended product.

The IR spectrum of this compound was in agreement with that of the compound provided in Example 1.

The triterpenoid saponin according to the present invention is a physiologically active substance that inhibits the absorption of glucose through intestines and prevents a rapid increase in the blood sugar level and has neither astringency nor bitterness.

Therefore, when the compound is added to diets containing sugar (including starches), it is possible to provide diets having an inhibited glucose absorption, so that it is understood that, in the case of patients suffering from diabetes mellitus who are on a high blood sugar level due to failure of insulin secretion, the inhibition of glucose absorption through intestines protects insulin secretion cells of exhausted pancreas, which is useful for treating and preventing diabetes mellitus.

The inhibitory effect of the triterpenoid saponin of the present invention on glucose adsorption will now be demonstrated.

1 mg of the triterpenoid saponin of the present invention prepared in Example 1 and 50 mg of CMC were added to physiological saline to provide a suspension which was orally administered to ddY mice at a dose of 0.5 mg/kg of the triterpenoid saponin. 60 min after the administration of the suspension, 1 g/kg of sucrose was orally administered. Thereafter, blood was drawn from veins of the fundus oculi 15 min, 30 min, 60 min and 120 min after the administration of the sugar to determine the blood glucose level.

The results are given in Fig. 2.

The ddY mice were 6 to 8 week-old male mice having a weight of 25 to 28 g (n = 10) and subjected to fasting from 24 hr before the initiation of the test. In the jejunum, the blood sugar level was 65 ± 5 mg/dl.

From the above results, it is apparent that the above-described triterpenoid saponin is useful as a preventive or therapeutic agent for diabetes mellitus. A toxic test for the triterpenoid saponin of the present invention will now be described.

Example 3

Tablets having the following composition per tablet were prepared.

| | |
|---|---|
| Triterpenoid saponin prepared in Example 1 | 10 mg |
| Extract of glycyrrhiza (corrigent) | 45 mg |
| Dextrin (filler) | 55 mg |
| Crystalline cellulose (binder) | 55 mg |
| CMC calcium (disintegrator) | 33 mg |
| Calcium stearate (lubricant) | 0.6 mg |

Acute toxicity test:

An acute toxicity test was effected by the following procedure. As a result, no toxicity was observed.

1. Test animal

Use was made of DDF male mice having a weight of 25 g, and each group consisted of 10 animals. The animals were ingested a solid feed (for breeding) while they freely got tap water as drinking water.

2. Sample

The triterpenoid saponin was suspended in 1% CMC physiological saline to a concentration of 200 mg/ml.

3. Test method

The test substance was administered with a probe by forced oral administration. The dose was 5000 mg/kg for one group and 2500 mg/kg for another group. Test period was 7 days. After the administration, the animals were continuously observed for 3 hr, and, thereafter, observation was effected of general symptoms every 6 hr.

The results are given in Table 2.

Table 2

| Dose (mg/kg) | Number of animals per group | Number of dead animals | Other general symptoms |
|---|---|---|---|
| 5000 | 10 | 0 | None of the animals exhibited any abnormal symptom. |
| 2500 | 10 | 0 | Do. |
| 0 | 10 | 0 | Do. |

Example 4

A sweet taste response inhibition test was effected for the triterpenoid saponin of the present invention and Gymnemic acid 1 and Gymnemic acid 2 obtained from Gymnema sylvestre, R. Br. known to have an inhibitory effect on glucose adsorption.

Reagents

The triterpenoid saponin prepared in Example 1 and Gymnemic acid 1 and Gymnemic acid 2 were dissolved in a 0.01 M/liter aqueous sodium bicarbonate ($NaHCO_3$) solution to prepare a 0.08% triterpenoid saponin solution, a 0.08% Gymnemic acid 1 solution and a 0.08 % Gymnemic acid 2 solution.

Separately, 0.1 M/liter, 0.2 M/liter, 0.3 M/liter, 0.4 M/liter and 0.5 M/liter aqueous sucrose solutions were previously prepared. The sweet taste response inhibition test was effected according to the following procedure.

Test procedure

1) 5 ml of the triterpenoid saponin solution or Gymnemic acid 1 or 2 solution was kept in mouth so that it was spread in the whole mouth.
2) 2 min after that, the sample solution was spit out, and the mouth was sufficiently rinsed with distilled water.
3) The above-described aqueous sucrose solutions were successively kept in the mouth in the order of concentration from the lowest to the highest, and the sucrose concentration of the solution which was first sweet to the taste was recorded.

The results are given in Table 3.

Table 3

| Sample solution | Aqueous Sucrose Solution Concentration (M/liter) 0.1 ①②③④⑤ | 0.2 ①②③④⑤ | 0.3 ①②③④⑤ | 0.4 ①②③④⑤ | 0.5 ①②③④⑤ |
|---|---|---|---|---|---|
| Control | ○○○○○ | | | | |
| Gymnemic acid 1 | ×××× × | ○×△×△ | ○○○×○ | ○○○△○ | ○○○○○ |
| Gymnemic acid 2 | ××××× | ××△×× | ×△△×× | ○○○○○ | |
| Compound of Invention | △○○△○ | ○○○○○ | | | |

Note) ○: Sweet to the taste
×: Not sweet to the taste
Δ: No clear taste
Control: 0.01 M/liter $NaHCO_3$ solution alone

From the results of Table 3, it is apparent that Gymnemic acid 1 exhibited the inhibitory effect on the sweet taste responses with respect to aqueous sucrose solutions having a sucrose concentration up to 0.2 M/liter, and Gymnemic acid 2 exhibited the inhibitory effect on the sweet taste responses with respect to aqueous sucrose solutions having a sucrose concentration up to 0.3 M/liter, whereas the triterpenoid saponin according to the present invention exhibited no inhibitory effect on the sweet taste responses.

The triterpenoid saponin used in the present invention has neither astringency nor bitterness and inhibits glucose adsorption, which renders the triterpenoid saponin useful as an additive to diets.

Gymnemic acid 1 is a compound represented by the formula II wherein R is a group represented by the formula III, and Gymnemic acid 2 is a compound represented by the formula II wherein R is a group represented by the formula IV.

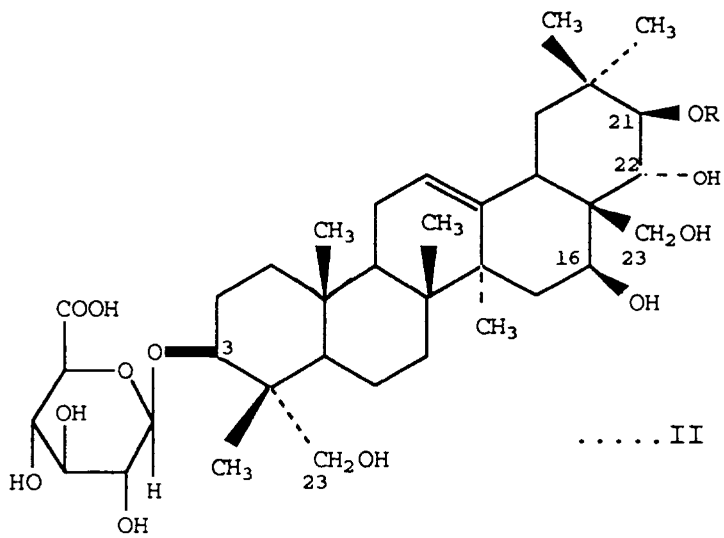

..... II

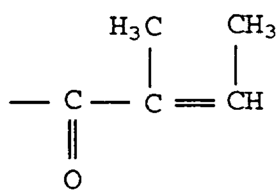

.... III

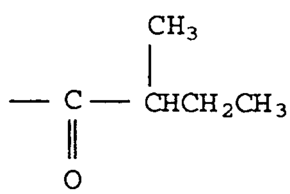

.... IV

The present invention will now be described in more detail with reference to the following Examples (formulation examples) and Comparative Examples, though it is not limited to these Examples only. In the following Examples, use was made of the triterpenoid saponin prepared in Example 1.

Example 5 (Formulation of Chocolate)

| | |
|---|---|
| Bitter | 40 g |
| Sucrose | 50 g |
| Cacao butter | 10 g |
| Triterpenoid saponin | 30 mg |

Comparative Example 1

For comparison, a chocolate was produced according to the same formulation as that of Example 5, except that Gymnemic acid extracted from Gymnema sylvestre, R. Br. was incorporated instead of the triterpenoid saponin in the same proportion as that of the triterpenoid saponin used in Example 5.

The chocolate prepared in Comparative Example 1 had astringency, and was not sweet to the taste. By contrast, the taste of the chocolate prepared in Example 5 was quite the same as a chocolate to which no triterpenoid saponin had been added.

Example 6 (Formulation of Rice Cake Stuffed with Bean Jam)

| | |
|---|---|
| Glutinous rice | 50 g |
| Salt | 0.1 g |
| Adzuki-bean gruel mixed with 16 g of granulated sugar and 30 mg triterpenoid saponin | 50.2 g |

The triterpenoid saponin may be contained in glutinous rice (skin).

Example 7 (Formulation of Noodles)

| | |
|---|---|
| Wheat flour | 100 g |
| Salt | 4 g |
| Water | 40 ml |
| Strong flour | q.s. |
| Triterpenoid saponin | 15 mg |

Example 8 (Formulation of Café Au Lait)

| | |
|---|---|
| Sucrose | 11.9 g |
| Glucose | 5.0 g |
| Glycinin | 0.2 g |
| Skimmilk powder | 8.0 g |
| Caramel | 0.5 g |
| Extract of coffee | 1.0 g |
| Coffee flavor | 0.05 ml |
| Triterpenoid saponin | 15 mg |

Water was added to the above-described ingredients to provide 180 ml of café au lait.

Example 9 (Formulation of Candy)

| | |
|---|---|
| Sucrose | 50 g |
| Viscous starch jelly | 50 g |
| Tartaric acid | 0.15 g |
| Citric acid | 0.35 g |
| Food dye | q.s. |
| Fruit oil | 0.1 ml |
| Triterpenoid saponin | 30 mg |

A candy was prepared according to the above-described formulation by a conventional method.

As with the chocolate prepared in Example 5, the products prepared in Examples 6 to 9 exhibited no abnormal taste.

As is apparent from the foregoing description, the present invention can provide a triterpenoid saponin having neither astringency nor bitterness but having an inhibitory effect on glucose adsorption, which triterpenoid saponin is useful as an additive to diets or a therapeutic agent for diabetes mellitus.

## Claims

1. A triterpenoid saponin represented by the following general formula I:

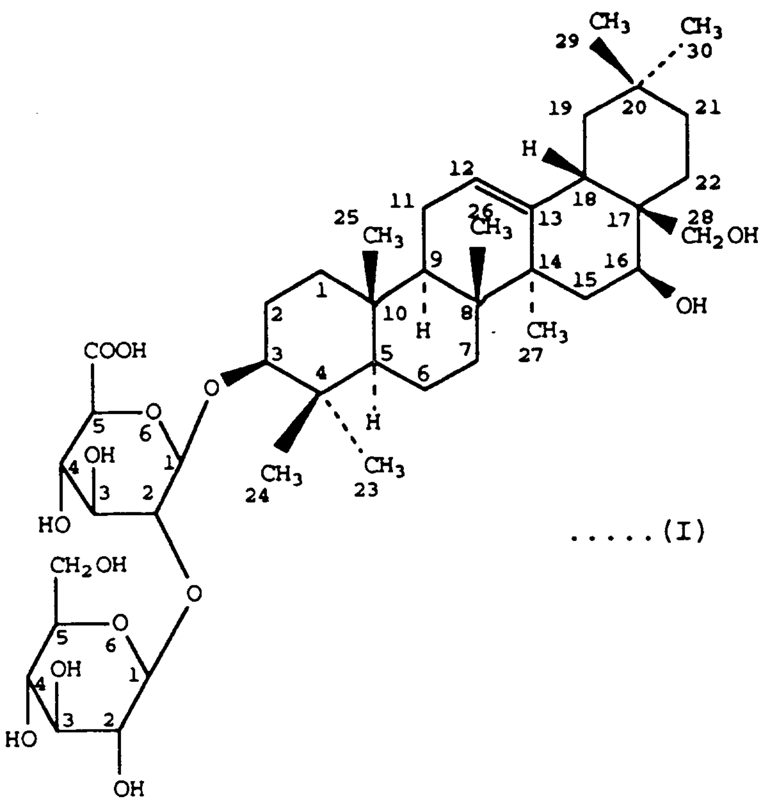

.....(I)

2. A compound according to claim 1 when substantially isolated from components of its natural environment.

3. A compound according to claim 1 or claim 2 obtainable by a process comprising subjecting leaves of Gymnema inodorum to extraction with a solvent, filtering the extract, evaporating the filtrate to dryness, washing the residue with petroleum ether, extracting the washed residue with acetone, filtering the extract, evaporating the filtrate to dryness, extracting the residue with diethyl carbonate, and precipitating a crystal from the extract.

4. A process for producing a triterpenoid saponin according to claim 1, comprising subjecting leaves of Gymnema inodorum to extraction with a solvent, filtering the extract, evaporating the filtrate to dryness, washing the residue with petroleum ether, extracting the washed residue with acetone, filtering the extract, evaporating the filtrate to dryness, extracting the residue with diethyl carbonate, and precipitating a crystal from the extract.

5. A triterpenoid saponin according to any of claims 1 to 3 for use as an active therapeutic substance.

6. A composition for preventing or treating diabetes mellitus, comprising as an active ingredient a triterpenoid saponin according to any of claims 1 to 3.

7. A composition according to claim 6 including an excipient

8. A composition according to claim 6 or 7 in the form of an additive to a diet, a diet, a confectionery, a beverage, a seasoning or a sweetener.

9. A composition according to claim 8 comprising a glucide.

10. A composition according to claim 9 comprising the saponin in an amount in the range 1 to 300 mg, preferably 10 to 90 mg, per 50 to 60g glucide.

*Fig. 1*

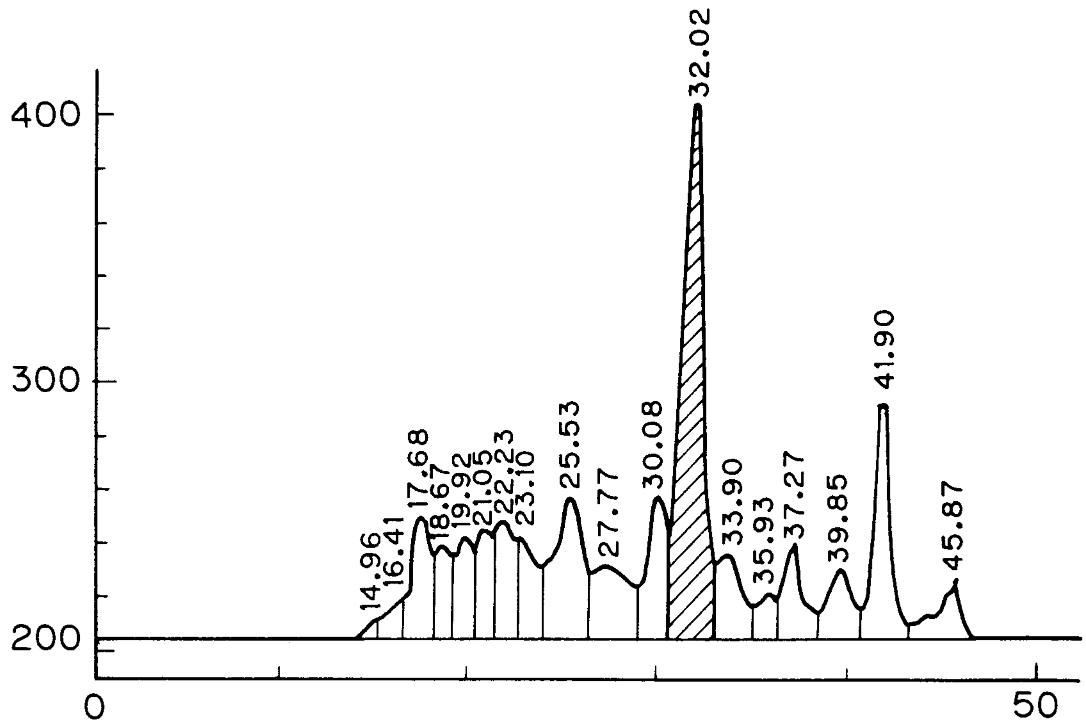
400
300
200
0
50
14.96
16.41
17.68
18.67
19.92
21.05
22.23
23.10
25.53
27.77
30.08
32.02
33.90
35.93
37.27
39.85
41.90
45.87

*Fig. 2*

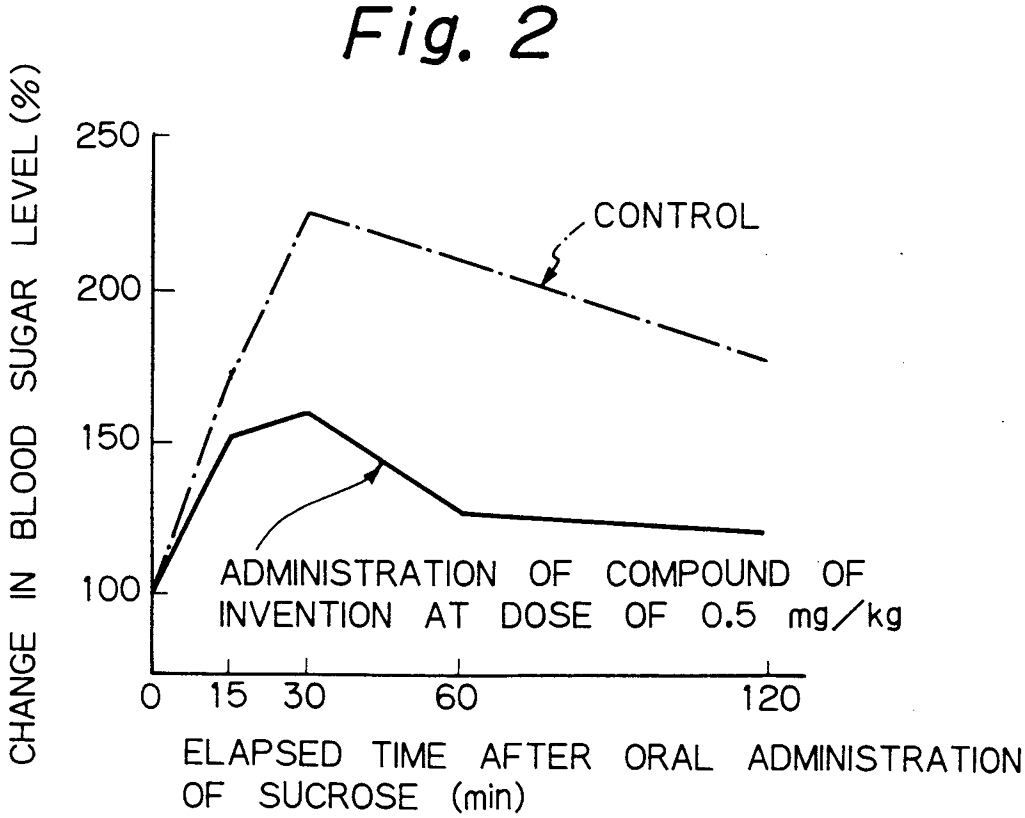
CHANGE IN BLOOD SUGAR LEVEL (%)
250
200
150
100
CONTROL
ADMINISTRATION OF COMPOUND OF INVENTION AT DOSE OF 0.5 mg/kg
0
15
30
60
120
ELAPSED TIME AFTER ORAL ADMINISTRATION OF SUCROSE (min)

European Patent Office

# EUROPEAN SEARCH REPORT

Application Number
EP 93 30 5973

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | DATABASE WPI<br>Section Ch, Week 8045,<br>Derwent Publications Ltd., London, GB;<br>Class B04, AN 80-79615<br>H. FUJIMARA 'Antidiabetic Agent Containing Ginseng Extract Prepared by Extraction with Water Followed by Extraction with Butanol or THF'<br>& JP-A-55 122 724 (SEISAN KAIHATSU KAGAKU) 21 September 1980<br>* abstract *<br>--- | 1-3,5-10 | C07J63/00<br>A61K31/58 |
| Y | EP-A-0 406 516 (DONG KOOK PHARMACEUTICAL CO., LTD.)<br>* whole document, particularly page 3, compounds (C) and (D) *<br>--- | 1-3,5-10 | |
| A | US-A-3 671 532 (G. CARRAZ ET AL)<br>* the whole document *<br>--- | 1,5 | |
| A | US-A-4 985 248 (Y. LIU)<br>* the whole document *<br>--- | 1,5 | TECHNICAL FIELDS SEARCHED (Int.Cl.6) |
| A | EP-A-0 515 755 (KOWA CHEMICAL INDUSTRIES CO., LTD)<br>* the whole document *<br>----- | 1,5 | C07J<br>A61K |

The present search report has been drawn up for all claims

| Place of search | Date of completion of the search | Examiner |
|---|---|---|
| THE HAGUE | 4 January 1994 | Watchorn, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)